# EUROPEAN PATENT SPECIFICATION

(11) **EP 0 729 943 B1**
(45) Date of publication and mention of the grant of the patent: **09.01.2002**
(21) Application number: 96200499.0
(22) Date of filing: 27.02.1996
(51) Int. Cl.: C07D 201/08

(54) **Process for the preparation of epsilon-caprolactam and epsilon-caprolactam precursors**
Verfahren zur Herstellung von Epsilon-Caprolactam und Epsilon-Caprolactam-Vorläufern
Procédé pour la préparation d'epsilon-caprolactame et de ses précurseurs

(30) Priority: 01.03.1995 US 396240
(43) Date of publication of application: 04.09.1996
(73) Proprietor: DSM N.V., 6411 TE Heerlen (NL); E.I. DU PONT DE NEMOURS AND COMPANY, Wilmington Delaware 19898 (US)
(72) Inventor: Wolters, Henricus Franciscus Wilhelmus, 6101 BT Echt (NL); Lane, Samuel Livingstone, Beaumont, Texas 77708 (US); Buijs, Wim, 6365 BM Schinnen (NL); Herkes, Frank Edward, Wilmington, Delaware 19808 (US); Haasen, Nicolaas Franciscus, 6141 MB Limbricht (NL)
(74) Representative: Kleiborn, Paul Erik

(56) References cited:
- EP-A- 0 376 122
- EP-A- 0 545 150
- DE-A- 3 602 375
- DE-A- 3 602 376
- DE-A- 3 602 377

## Description

The invention relates to a process for the preparation of ε-caprolactam and ε-caprolactam precursors, starting from 5-formylvalerate ester, ammonia and hydrogen in the presence of a hydrogenation catalyst.

ε-Caprolactam precursors are here defined as 6-aminocaproate ester, 6-aminocaproic acid and 6-aminocaproamide.

Such a process is known from US-A-4730040. According to US-A-4730040, a mixture of ε-caprolactam and 6-aminocaproate esters are prepared by contacting 5-formylvalerate esters with ammonia, hydrogen a hydrogenation catalyst and methanol as solvent.

A drawback of this known process is that a relatively large amount of by-products are formed, decreasing the yield of 6-aminocaproate ester and ε-caprolactam.

By-products are for example 6-hydroxycaproate esters, secondary amines, tertiary amines and Schiff bases.

The aim of the present invention is to provide a process wherein less of these by-products are formed and wherein a higher yield of ε-caprolactam and ε-caprolactam precursors is achieved.

The object of this invention is achieved in that in a first step 5-formylvalerate ester is reacted with ammonia under non-hydrogenating conditions and in a second step the reaction product obtained in the first step is converted to ε-caprolactam and ε-caprolactam precursors under hydrogenating conditions in the presence of ammonia.

It has been found that if the process is performed according to the invention, less by-products are formed, especially 6-hydroxycaproate esters and secondary amines. An additional advantage is that the yield remains constant over a longer period of time when the process is carried out continuously. This is in contrast to when the process of US-A-4730040 is performed continuously over a longer period of time. Within several hours of operation the yield of this process has been found to decrease significantly.

DE-A-3602375 and DE-A-3602376 describe a process for the preparation of 6-aminocaproate ester and ε-caprolactam by contacting methyl-5-formylvalerate with an excess of ammonia, hydrogen, a hydrogenation catalyst and methanol as solvent. These processes do not include a step of reaction between 5-formylvalerate ester and ammonia carried out in non-hydrogenating conditions.

5-Formylvalerate ester can be represented by the following general formula: 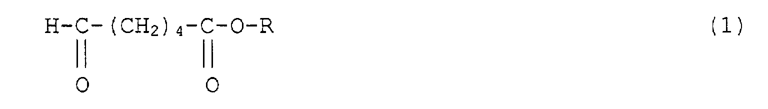 where R comprises a (cyclo)alkyl, an aryl or an aralkyl with 1 to 20 carbon atoms. Examples of such groups are: methyl, ethyl, propyl, isopropyl, n-butyl, tert-butyl, isobutyl, cyclohexyl, benzyl and phenyl. By preference, methyl or ethyl are used.

The first step is carried out under non-hydrogenating conditions. By 'non-hydrogenating conditions' it is understood in this invention that the reaction conditions are such that, no hydrogen is present or if hydrogen is present, the 5-formylvalerate esters or a reaction product of it is not or virtually not reduced by the hydrogen. In general, non-hydrogenating conditions are achieved by carrying out the first step in the absence of a hydrogenation catalyst.
In one such an embodiment of the process the hydrogen can already be present in the first step. If the hydrogenation catalyst is already present in the first step, a situation of non-hydrogenating conditions can also be realized by not adding hydrogen to the reaction mixture until after completing the first step.

It has been found that the best results with regard to yield to 6-aminocaproate ester are achieved when the conversion of 5-formylvalerate ester in the first step is more than 90%, preferably more than 99%. Too low of a conversion will result in an increase of, for example, the 6-hydroxycaproate ester and/or secondary amines formation. It has further been found that this yield will be negatively influenced when the residence or contact time in the first step is greater than the residence time or contact time needed for a virtually complete conversion of the 5-formylvalerate ester. An excessively long residence or contact time causes increased formation of by-products other than 6-hydroxycaproate ester. Accordingly, it is preferred to avoid any "overreacting" in the first step.

The temperature in the first step may be up to 120°C and is preferably between 0 and 100°C. The temperature is not very critical within this range. In a large scale operation the temperature will preferably be above 10°C and more preferably above 20°C, because then no excessive cooling will be required. When the first step is performed in a continuously operated tube reactor the feed temperature may be for example, around 10°C. Because of the heat of the exothermic reaction the temperature of the reaction mixture may rise so that at the end of the reactor a higher outlet temperature is achieved.

As explained above a too low contact or residence time and a too high contact or residence time in the first step will result in by-product formation. The optimal residence or contact time at which the conversion of 5-formylvalerate ester is virtually completed will depend on the reaction conditions, for example temperature, concentration of reactants and method of mixing. The optimal residence time or contact time can be easily determined by the man skilled in the art. Starting from the temperature and concentration range here described the residence or contact time will under normal mixing condition normally be between several seconds and 2 minutes. Preferably the contact or residence time is higher than 5 seconds.

The first step is carried out in the presence of ammonia, preferably a molar excess of ammonia being chosen such that the molar ratio ammonia and 5-formylvalerate esters is between about 500:1 and about 1:1 calculated from the starting amount of 5-formylvalerate ester.

The pressure in the first step is not critical. If the first step is carried out in the absence of hydrogen, the pressure in general is between atmospheric pressure and 12 MPa, depending on the temperature and the composition of the reaction mixture that have been chosen. In a continuous process the pressure in the first and second step will be about the same.

The first step can be carried in the presence of a catalyst, for example an acid ion exchanger or an acidic metal oxide catalyst, for example alumina or TiO₂. The conversion of 5-formylvalerate ester in the first step also proceeds favorably in the absence of a catalyst. Because the overall yield to 6-aminocaproate ester is not greatly influenced by the presence of a catalyst in the first step, such a catalyst is generally not used.

The reaction product obtained in the first step is converted in the second step, completely or partly to ε-caprolactam and ε-caprolactam precursors, under hydrogenating conditions in the presence of ammonia. By preference the second step is carried out in the presence of an excess of ammonia such that the molar ratio ammonia and 5-formylvalerate ester is between 500:1 and about 1:1 calculated from the starting amount of 5-formylvalerate ester (used in the first step).

The term 'hydrogenating conditions' means that the reaction conditions are such that the reaction product(s) obtained in the first step can be reduced by hydrogen. In general hydrogenation conditions are achieved when hydrogen and a hydrogenation catalyst are present.

The molar quantity of hydrogen that is needed for the second step is at least equal to the molar quantity of the 5-formylvalerate ester which was started from in the first step. By preference, however, a 1.01 to hundred fold molar excess of hydrogen is applied in the second step.

The total pressure used in the second step is preferably between 0.5 and 20 MPa. It has been found that the efficacious pressure in the second step will also depend on the optional solvent used. The man skilled in the art can readily ascertain this optimum with a limited set of experiments.

In the process according to the invention, at least one step may be carried out in the presence of an additional solvent. Ammonia may also serve as the solvent of the process. If an additional solvent is used preferably the second step and more preferably both steps are performed in the presence of the additional solvent. Suitable solvents are water, tertiary amines, ethers, alcohols with 1-6 carbon atoms, ethers with 2 to 10 carbon atoms, for example methyl-tertiary butyl ether and tertiary amylmethyl ether or high boiling organic sovlents, for example a high boiling paraffin solvent. Preferably, an alcohol, an ether or water is used as the additional solvent. From the alcohols the one corresponding to the R-group (formula (1)) of the 5-formylvalerate ester is preferred. Water and the corresponding alcohol are preferred solvents because these compounds are also formed as a reaction product in the process according to the invention. Examples of these are methanol, ethanol, propyl alcohol, isopropyl alcohol, n-butyl alcohol, tert-butyl alcohol, isobutyl alcohol, cyclohexyl alcohol, benzyl alcohol and phenol or mixtures thereof. Most preference is given to methanol and ethanol. Mixtures of additional solvents may also be used in the process according to the invention. Examples of such mixtures are water-methanol, water-ethanol or methylethanol.

Most preferably water or water-corresponding alcohol mixtures are used as solvent in both steps. These water-corresponding alcohol mixtures are advantageous because the 5-formylvalerate ester has increase solubility in these mixtures compared to pure water. Water is advantageous because it can also be used as solvent in a cyclization step following the process according to the invention. In such a cyclization step the ε-caprolactam precursors are converted to ε-caprolactam at elevated temperature as for example described in US-A-3485821. When using water as solvent in the process according to the invention the reaction mixture obtained can be directly used in such a cyclization.

The second step is carried out at a temperature which in general is between 40 and 200°C, more preferably between 70 and 180°C and most preferably between 80 and 160°C.

The residence or contact time in the second step should be high enough to reduce virtually all the intermediate products formed in the first step. Higher residence time or a longer contact time will result in that more ε-caprolactam is formed. The residence or contact time is preferably between around a half minute to a couple of hours. When the process is carried out batch wise or in a continuously operated slurry reactor the contact or residence time respectively will generally be higher than the residence time when a continuously operated tube reactor is used.

The hydrogenation catalyst comprises at least one of the metals choosen from the metals of Groups 8-10 of the Periodic System of the Elements (Handbook of Chemistry and Physics, 70th edition, CRC Press, 1989-1990). Preference is given to Ru-, Ni- or Co-containing catalysts. In addition to Ru, Co and/or Ni the catalysts can also contain other metals for example Cu, Fe and/or Cr. The catalytically active metals may be applied onto a carrier or not. Suitable carriers are for example aluminium oxide, silica, titanium oxide, magnesium oxide, zirconium oxide and carbon. Non-carried metals can be used for example in the form of a finely dispersed suspension for example finely dispersed ruthenium. Preferred Ni- and Co-containing catalysts are Raney nickel and Raney cobalt optionally in combination with small amounts of another metal, for example Cu, Fe and/or Cr.

It has been found that the yield of ε-caprolactam and ε-caprolactam precursors has an optimum which also depends on reaction conditions of the second step. These reaction conditions are for example temperature, pressure, ammonia/5-formylvalerate ratio and the presence or absence of an additional solvent. The conditions to obtain the optimum yield can easily be found by the man skilled in the art within the conditions as outlined above.

The two step process according to the invention can be performed batch wise or continuous. A large scale commercial process will preferably be performed continuously. For the first step, it is important that the reactants are sufficiently contacted at a certain temperature during a specified period of time as described above. Any manner of contacting will usually suffice. For example a tube reactor with or without, for example, internal baffling or packing or a static mixer is a possible contacting unit for the first step. To control the temperature in the first step it may be advantageous to use cooling devices, for example cooled walls or a cooling spiral placed in the contacting unit.

The second step may be performed in a fixed bed reactor in which for example the heterogeneous hydrogenation catalyst is present. An advantage of this reactor is that the reactants are easily separated from the hydrogenation catalyst. Another manner of operating the second step is by way of one or more continuously well mixed contactors in series in which the hydrogenation catalyst is present as a slurry (slurry reactor). This manner of operation has the advantage that the heat of reaction can be easily controlled by for example a cooled feed or by way of internally placed cooling devices. An example of a specific and suitable slurry reactor is a one or multiple staged bubble column or a gas lift-loop reactor.

The concentration of 5-formylvalerate ester and the products of the process (ε-caprolactam and ε-caprolactam precursors) in the reaction mixture in both steps is preferably between 1 and 50 wt.% and more preferably between 10 and 35 wt.%.

The yield in the Examples I-V were calculated as mol product relative to mol starting 5-formylvalerate. A practically 100% conversion of 5-formylvalerate was achieved in all Examples.

### Example I

Preparation of methyl 6-aminocaproate and ε-caprolactam from methyl 5-formylvalerate (M5FV) without using an additional solvent.

Two tubular reactors were arranged in series. The first reactor consisted of a 150 mm long tube with a diameter of 3.2 mm. Ammonia and M5FV were fed at room temperature. The ammonia/M5FV molar ratio was 28:1. M5FV was supplied at a rate of 40 l/h (ammonia: 132 g/h). The residence time in this reactor was 17 seconds (sec.). The feed streams of the first reactor have a temperature of 20°C. No measures were taken to control the temperature in the first reactor. The outlet temperature was 77°C.

The second reactor consisted of a 430 mm long vertical tube with a diameter of 8 mm. The reactants were pumped upward through the reactor. This reactor was heated over its full length. The second reactor was filled with 10 g of an activated catalyst comprising 1 wt.% Ru on aluminum oxide. The empty volume above the catalyst bed was filled with glass beads. The total pressure was 9.6 MPa. The hydrogen flux was 35 normal l/h. The inlet temperature of the second reactor was 77°C and the outlet temperature in the second reactor was 127°C. The liquid residence time was 3 minutes. The experiment was run for 2 hours in which the yields remained constant during this period.
The molar yields determined by means of Gas Chromatography-mass Spectrometry (GC-MS), were 85% of methyl 6-aminocaproate and 4% of ε-caprolactam.

### Example II

Example I was repeated in which a stream of M5FV solution (20% by weight M5FV in methanol) at a rate of 210 ml/h and 96 gr/h ammonia was pumped together with a stream of hydrogen at a rate of 20 standard l/h through the two reactors. The total pressure was 9.2 MPa.

The first reactor was kept at 20°C by using a cooling coil. The temperature in the second reactor was maintained at 129°C.

The residence time in the first reactor was 12 seconds and in the second reactor 130 seconds. The molar yields based on M5FV turned out to be 96.5% by weight methyl 6-aminocaproate and 3.4% by weight ε-caprolactam. The experiment was run for 4 hours in which the above results remained constant during this period.

### Example III

Example II was repeated in which 18% by weight solution of M5FV in methanol was used.
The solution of M5FV in methanol was fed at a rate of 210 ml/h into the first one of the two reactors together with ammonia at a rate of 65 gr/h. Both liquids were fed at room temperature.
The yield based on M5FV turned out to be 97.5% methyl 6-aminocaproate and 2.5% ε-caprolactam.

### Comparative experiment A

Preparation of methyl 6-aminocaproate from M5FV without the first reactor using methanol as solvent.

Ammonia, a 16.8 wt.% mixture of M5FV in methanol and hydrogen were directly fed to the bottom of the second reactor filled with 1% Ru on aluminum oxide (Johnson Matthey).
The ammonia/M5FV molar ratio was 13.5:1. Hydrogen was pumped through the reactor at a rate of 20 l/hr. The total pressure was 10 MPa. The temperature was set at 129°C.
The yields, based on the converted M5FV turned out to be 83% methyl-6-aminocaproate, 2% 6-aminocaproic amide and 2% ε-caprolactam. The total conversion after 1 hour was 92%. The conversion after 2 hours was 70% and after 3 hours only 48%. The selectivity, the total amount of 6-aminocaproic amide, ε-caprolactam and methyl 6-aminocaproate with respect to the total amount of converted M5FV went down from 87% after 1 hour to 35% after 3 hours.

### Example IV

A vertically placed fixed-bed reactor (diameter = 8 mm, total length = 43 cm and fill height = 30 cm) with electrical heating was filled with 10.3 g of an activated catalyst consisting of ruthenium on an alumina carrier (3 wt.% ruthenium; diameter of catalyst particles was 1 mm). An hourly supply of 20 Nl hydrogen (0.8 mol), 140 ml of a 9 wt.% solution of methyl 5-formylvalerate in methyl tert-butyl ether and 116 ml (4 mol) of liquid ammonia was fed to this reactor. Prior to feeding the reactants to the fixed bed reactor the reactants were continuously mixed in a first step at 20°C and 9.6 MPa in a volume of approximately 7 ml (residence time of approximately 100 seconds). The resulting mixture was passed over the catalyst from bottom to top at a temperature of 130°C and a pressure of 9.6 MPa (residence time approximately 1.5 minutes). The reaction mixture was discharged from the top of the fixed-bed reactor and after cooling the overpressure was let off. From quantitative High Pressure Liquid Chromatography (HPLC) analysis it appeared that all the methyl 5-formylvalerate had been converted and that the molar yield of methyl 6-aminocaproate was 88%, while the yield of ε-caprolactam was 10%, relative to the quantity of starting methyl 5-formylvalerate. So the combined yield of methyl 6-aminocaproate and ε-caprolactam together was 98%.

### Example V

Example IV was repeated in which the same volume of liquid ammonia was used instead of methyl tert-butyl ether. The yield of methyl 6-aminocaproate and ε-caprolactam was 94%. All of the methyl 5-formylvalerate had been converted.

### Example VI

At a pressure of 5.0 MPa, 45 g/hr of methyl-5-formylvalerate, 495 g/hr of water and 360 g/hr of ammonia was pumped through a tube which was cooled by a water bath so that a constant temperature of 35°C was maintained in the tube. Almost no back mixing occurred and the (liquid) residence time was 15 seconds. The resulting mixture leaving the tube (first step) was fed to a continuously stirred tank reactor (CSTR), a Hastelloy C autoclave of 1 liter liquid volume. The reactor was stirred at 1250 rpm. The residence time was 60 minutes and the temperature was kept at 100°C. To the reactor 10 g/hr of hydrogen was fed. The reactor was filled with 50 g of an unpromoted Raney Nickel catalyst (93 wt.% nickel and 7 wt.% aluminum, average particle size: 30 µm from Activated Metals Company (A5000)).

The effluent was analyzed by HPLC after 3 and 6 hours of operation. The results are summarized in Table 1. The yields after 3 and 6 hours of operation were comparable and within their respective error range. The conversion of methyl 5-formylvalerate was 100%.

**TABLE 1**

| | 3 hours | 6 hours |
|---|---|---|
| 6-aminocaproamide | 58.7 (1) | 59.8 |
| 6-aminocaproic acid | 23.9 | 25.3 |
| methyl-6-aminocaproate | 0.4 | 0.0 |
| ε-caprolactam | 17.0 | 14.9 |

| | | |
|---|---|---|
| (1) Results in weight percentage. | | |

As is clear from Table 1 high yields, up to a total of 100%, are obtained of ε-caprolactam and ε-caprolactam.

### Example VII

Example VI was repated for 50 hours at a pressure of 3 MPa, in which 81.7 g/hr of methyl-5-formylvalerate (M5FV), 203 g/hr ammonia and 526 g/hr water was pumped through the tube at a temperature of 35°C. Almost no back-mixing ocurred in the tube and the liquid residence time in the tube was 15 seconds. The reaction mixture leaving the tube contained no M5FV.

The mixture leaving the tube was fed to the continuously stirred tank reactor (CSTR) in which a liquid hold-up of 1 liter of liquid was maintained. The catalyst in the CSTR was a 5wt% ruthenium on Al₂O₃ catalyst (Engelhard: ESCAT 44) and the catalyst concentration was maintained at 103 g/l. The CSTR was stirred at 1260 rpm. The pressure in the CSTR was kept constant at 3 MPa and the temperature at 120°C. The residence time was 60 minutes. To the reactor a net amount of 5.0 g/hr of hydrogen was fed.

The effluent of the CSTR was analyzed by High Pressure Liquid Chromotography (HPLC) every 4 hours. The composition of the effluent did not vary significantly during the 50 hours of operation. The average composition of the effluent in the last 28 hours was 28 mol% 6-aminocaproic acid (6ACA), 47.2 mol% 6-aminocaproic acid amide (6ACAM), 24.2 mol% ε-caprolactam (CAP), 0.6 mol% methyl 6-aminocaproate (M6AC). Thus a 100 mol% yield to ε-caprolactam and ε-caprolactam precursors is obtained.

### Example VIII

Example VII was repeated for 22 hours, in which the water feed was replaced by a mixture of water and methanol (15 wt% methanol). The feed rate of this mixture was 511 g/hr. Almost no back-mixing ocurred in the tube and the liquid residence time in the tube was 15 seconds. The reaction mixture leaving the tube contained practically no methyl-5-formylvalerate.

The catalyst concentration in the CSTR was 96.0 g/l.

The composition of the effluent did not vary significantly during the 22 hours of operation. The average composition of all the products formed in the last 12 hours was 22.5 mol% 6ACA, 48.0 mol% 6ACAM, 27.4 mol% ε-caprolactam, 2.1 mol% methyl 6-aminocaproate. Thus a 100 mol% yield to ε-caprolactam and ε-caprolactam precursors is obtained.

## Claims

1. Process for the preparation of ε-caprolactam and ε-capralactam precursors selected from 6-aminocaproate ester, 6-aminocaproic acid and/or 6-aminocaproamide, starting from 5-formylvalerate ester, wherein in a first step 5-formylvalerate ester is reacted with ammonia in the absence of hydrogen and/or a hydrogenation catalyst, and in a second step the reaction product obtained in the first step is converted in the presence of ammonia, hydrogen and a hydrogenation catalyst to ε-caprolactam and the ε-caprolactam precursors.

2. Process according to claim 1, wherein 5-formyl-valerate ester is represented by the following general formula 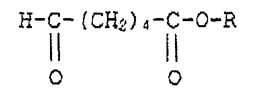 where R comprises a group containing from 1-20 carbon atoms and R is selected from a (cyclo)alkyl, an aryl or an aralkyl.

3. Process according to any one of claims 1-2, **characterized in that** the process is carried out in the presence of an ether with 2 to 10 carbon atoms.

4. Process according to claim 3, **characterized in that** methyl-tertiary butyl ether is used.

5. Process according to claim 2, wherein R is methyl or ethyl.

6. Process according to claim 2 or 5, **characterized in that** the first and second step are performed in the presence of a mixture of water and an alcohol according to ROH.

7. Process according to any one of claims 1-6, wherein the first step is carried out in the absence of a hydrogenation catalyst.

8. Process according to any one of claims 1-7, wherein more than 90% of the 5-formylvalerate ester is converted in the first step.

9. Process according to any one of claims 1-8, wherein the temperature in the first step is between 0-100°C.

10. Process according to any one of claims 1-9, wherein a molar excess of ammonia is present in the first and second step.

11. Process according to any one of claims 1-10, wherein the second step is carried out at a temperature between 70 and 180°C.

12. Process according to any one of claims 1-11, wherein the second step is carried out in the presence of a hydrogenation catalyst comprising at least one metal selected from Groups 8-10 of the Periodic System of the Elements.

13. Process according to claim 12, wherein the catalyst comprises at least one of the metals Ni, Co or Ru.

14. Process according to any one of claims 1-13, wherein the first and the second step are performed continuously.

## Patentansprüche

1. Verfahren zur Herstellung von ε-Caprolactam und ε-Caprolactam-Vorstufen, ausgewählt aus 6-Aminocapronsäureester, 6-Aminocapronsäure und/oder 6-Aminocaproamid, ausgehend von 5-Formylvaleriansäureester, wobei in einem ersten Schritt 5-Formylvaleriansäureester mit Ammoniak in Abwesenheit von Wasserstoff und/oder einem Hydrierungskatalysator umgesetzt wird und in einem zweiten Schritt das in dem ersten Schritt erhaltene Reaktionsprodukt in Gegenwart von Ammoniak, Wasserstoff und einem Hydrierungskatalysator zu ε-Caprolactam und den ε-Caprolactam-Vorstufen umgewandelt wird.

2. Verfahren nach Anspruch 1, wobei 5-Formylvaleriansäureester durch die nachstehende allgemeine Formel wiedergegeben wird 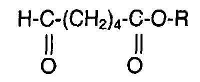 worin R eine Gruppe, enthaltend 1 bis 20 Kohlenstoffatome, umfasst und R aus einer (Cyclo)Alkyl-, einer Aryl- oder einer Aralkylgruppe ausgewählt ist.

3. Verfahren nach einem der Ansprüche 1 bis 2, **dadurch gekennzeichnet, dass** das Verfahren in Gegenwart eines Ethers mit 2 bis 10 Kohlenstoffatomen ausgeführt wird.

4. Verfahren nach Anspruch 3, **dadurch gekennzeichnet, dass** Methyl-tert.-butylether verwendet wird.

5. Verfahren nach Anspruch 2, wobei R Methyl oder Ethyl darstellt.

6. Verfahren nach Anspruch 2 oder 5, **dadurch gekennzeichnet, dass** der erste und zweite Schritt in Gegenwart eines Gemisches von Wasser und einem Alkohol gemäß ROH durchgeführt werden.

7. Verfahren nach einem der Ansprüche 1-6, wobei der erste Schritt in Abwesenheit eines Hydrierungskatalysators ausgeführt wird.

8. Verfahren nach einem der Ansprüche 1-7, wobei mehr als 90 % des 5-Formylvaleriansäureesters im ersten Schritt umgewandelt werden.

9. Verfahren nach einem der Ansprüche 1-8, wobei die Temperatur im ersten Schritt zwischen 0 bis 100°C liegt.

10. Verfahren nach einem der Ansprüche 1-9, wobei ein molarer Überschuss von Ammoniak im ersten und zweiten Schritt vorliegt.

11. Verfahren nach einem der Ansprüche 1-10, wobei der zweite Schritt bei einer Temperatur zwischen 70 und 180°C ausgeführt wird.

12. Verfahren nach einem der Ansprüche 1-11, wobei der zweite Schritt in Gegenwart eines Hydrierungskatalysators, umfassend mindestens ein Metall, ausgewählt aus den Gruppen 8-10 des Periodensystems der Elemente, ausgeführt wird.

13. Verfahren nach Anspruch 12, wobei der Katalysator mindestens eines der Metalle Ni, Co oder Ru umfasst.

14. Verfahren nach einem der Ansprüche 1-13, wobei der erste und der zweite Schritt kontinuierlich durchgeführt werden.

## Revendications

1. Procédé de préparation d'ε-caprolactame et de précurseurs d'ε-capro-lactame choisis parmi l'ester 6-aminocaproate, l'acide 6-aminocaproïque et/ou le 6-aminocaproamide, à partir d'un ester 5-formylvalérate, dans lequel, dans une première étape, l'ester 5-formylvalérate est mis à réagir avec de l'ammoniac en l'absence d'hydrogène et/ou de catalyseur d'hydro-génation et, dans une deuxième étape, le produit réactionnel obtenu dans la première étape est converti en présence d'ammoniac, d'hydrogène et d'un catalyseur d'hydrogénation en ε-caprolactame et en précurseurs d'ε-caprolactame.

2. Procédé selon la revendication 1, dans lequel l'ester 5-formylvalérate est représenté par la formule générale suivante : 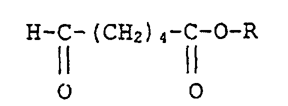 où R comprend un groupe contenant de 1 à 20 atomes de carbone et R est choisi parmi un (cyclo)alkyle, un aryle ou un aralkyle.

3. Procédé selon l'une quelconque des revendications 1 ou 2, **caractérisé en ce que** le procédé est conduit en présence d'un éther comprenant 2 à 10 atomes de carbone.

4. Procédé selon la revendication 3, **caractérisé en ce que** l'éther de méthyle et de butyle tertiaire est utilisé.

5. Procédé selon la revendication 2, dans lequel R est un méthyle ou un éthyle.

6. Procédé selon la revendication 2 ou 5, **caractérisé en ce que** la première et la seconde étape sont conduites en présence d'un mélange d'eau et d'un alcool en conformité avec ROH.

7. Procédé selon l'une quelconque des revendications 1 à 6, dans lequel la première étape est conduite en l'absence d'un catalyseur d'hydrogénation.

8. Procédé selon l'une quelconque des revendications 1 à 7, dans lequel plus de 90% de l'ester 5-formylvalérate est converti dans la première étape.

9. Procédé selon l'une quelconque des revendications 1 à 8, dans lequel la température, dans la première étape, est comprise entre 0 et 100°C.

10. Procédé selon l'une quelconque des revendications 1 à 9, dans lequel un excès molaire d'ammoniac est présent dans les première et seconde étapes.

11. Procédé selon l'une quelconque des revendications 1 à 10, dans lequel la seconde étape est conduite à une température comprise entre 70 et 180°C.

12. Procédé selon l'une quelconque des revendications 1 à 11, dans lequel la seconde étape est conduite en présence d'un catalyseur d'hydrogénation comprenant au moins un métal choisi dans les groupes 8 à 10 du tableau périodique des éléments.

13. Procédé selon la revendication 12, dans lequel le catalyseur comprend au moins l'un des métaux Ni, Co ou Ru.

14. Procédé selon l'une quelconque des revendications 1 à 13, dans lequel la première et la seconde étape sont conduites en continu.
